# EUROPEAN PATENT APPLICATION

(11) **EP 4 190 900 A1**
(43) Date of publication of application: **07.06.2023**
(21) Application number: 21849553.9
(22) Date of filing: 21.07.2021
(51) Int. Cl.: C12N 15/12, A01K 67/027, C07K 14/435, C12N 5/10, G01N 33/567

(54) **MUTANT INSECT OLFACTORY RECEPTOR PROTEIN**

(30) Priority: 27.07.2020 JP 2020126684
(71) Applicant: SUMITOMO CHEMICAL COMPANY, LIMITED, Tokyo 103-6020 (JP)
(72) Inventor: OZOE, Atsufumi, Osaka-shi, Osaka 554-8558 (JP); TAKAHASHI, Yasuhiko, Osaka-shi, Osaka 554-8558 (JP)
(74) Representative: Vossius & Partner Patentanwälte Rechtsanwälte mbB
(86) International application number: PCT/JP2021/027265
(87) International publication number: WO 2022/024902

(57) **Abstract**

An object is to provide a technique of increasing the response activity of an olfactory receptor to a chemical substance, and the object is achieved by introducing a mutation into a specific site in an insect olfactory receptor protein so as to satisfy a specific condition.

## Description

### Technical Field

The present disclosure relates to a mutant-insect olfactory receptor protein.

### Background Art

Odorants that characterize, for example, specific human diseases or mental status have been identified and are highly valuable as diagnostic markers. Thus, various odor sensors targeting these odorants have been actively developed. Because the olfactory receptors of creatures have excellent properties not found in conventional odor sensor elements such as semiconductors in terms of diversity, sensitivity, and selectivity, the development of new odor sensors using such olfactory receptors as sensor elements is awaited.

Because odor markers are generally low in concentration, it is desirable to prepare a highly active olfactory receptor. Current research reports that substitution of some amino acid sites in an olfactory receptor alters the selectivity for ions that transmit the receptor.

### Citation List

### Patent Literature

PTL 1: JP2013-27376A
PTL 2: JP2018-59786A
PTL 3: JP2012-78351A

### Non-patent Literature

NPL 1: PLoS ONE, March 2012, Volume 7, Issue 3, e32372.

### Summary of Invention

### Technical Problem

An object of the present disclosure is to provide a technique of increasing the response activity of an olfactory receptor to a chemical substance.

### Solution to Problem

The present inventors conducted extensive research to achieve the object and found that response activity to a chemical substance can be increased by introducing a mutation into a specific site in an insect olfactory receptor protein so as to satisfy a specific condition. The inventors conducted further research based on this finding and completed the invention in the present disclosure. Specifically, the present disclosure includes the following aspects.
Item 1. A mutant-insect olfactory receptor protein, comprising amino acid sequence B composed of amino acid sequence A containing a mutation, wherein
   the amino acid sequence A is in a corresponding wild-type insect olfactory receptor protein and represented by formula (1) :
      φ₇X₁X₂Z₁X₃X₄Z₂Z₃φ₆Uφ₅φ₄φ₃φ₂φ₁X₅X₆ wherein X₁ to X₆ and Z₁ to Z₃ represent amino acids derived from the amino acid sequence of the wild-type insect olfactory receptor, φ₁ represents a hydrophobic amino acid, φ₂ to φ₇ each independently represent an uncharged polar amino acid or a hydrophobic amino acid, and U represents an uncharged polar amino acid, and
   the mutant-insect olfactory receptor protein satisfies at least one condition selected from the group consisting of the following conditions 1 to 3:
      condition 1: in the amino acid sequence B,
         X₃ and/or X₄ is substituted, and
         X₃ is a branched-chain amino acid and/or X₄ is an uncharged polar amino acid,
      condition 2: if X₅ and X₆ in the amino acid sequence A are both a positively charged polar amino acid, X₆ in the amino acid sequence B is an amino acid other than a positively charged polar amino acid, and
      condition 3:
         if either X₁ or X₂ or both in the amino acid sequence A are a positively charged polar amino acid,
         in the amino acid sequence B,
            X₁ and/or X₂ is substituted, and
            X₁ is an uncharged polar amino acid and/or X₂ is a hydrophobic amino acid.
Item 2. The mutant-insect olfactory receptor protein according to Item 1,
   wherein
   the mutant-insect olfactory receptor protein satisfies condition 1, and
   X₃ represents isoleucine or leucine and/or X₄ represents serine or asparagine.
Item 3. The mutant-insect olfactory receptor protein according to Item 1 or 2,
   wherein
   the mutant-insect olfactory receptor protein satisfies condition 2, and
   X₆ represents an uncharged polar amino acid.
Item 4. The mutant-insect olfactory receptor protein according to any one of Items 1 to 3,
   wherein
   the mutant-insect olfactory receptor protein satisfies condition 2, and
   X₆ represents serine or threonine.
Item 5. The mutant-insect olfactory receptor protein according to any one of Items 1 to 4,
   wherein
   the mutant-insect olfactory receptor protein satisfies condition 3, and
   X₁ represents serine or glutamine and/or X₂ represents alanine.
Item 6. The mutant-insect olfactory receptor protein according to any one of Items 1 to 5,
   wherein
   in condition 1, X₃ represents isoleucine or leucine and/or X₄ represents serine or asparagine,
   in condition 2, X₆ represents serine or threonine, and
   in condition 3, X₁ represents serine or glutamine and/or X₂ represents alanine.
Item 7. The mutant-insect olfactory receptor protein according to any one of Items 1 to 6, which satisfies at least one condition selected from the group consisting of the following conditions 1A to 3A:
   condition 1A: in the amino acid sequence B, X₃ is a branched-chain amino acid and/or X₄ is an uncharged polar amino acid,
   condition 2A: in the amino acid sequence B, X₆ is an amino acid other than a positively charged polar amino acid, and
   condition 3A: in the amino acid sequence B, X₁ is an uncharged polar amino acid and/or X₂ is a hydrophobic amino acid.
Item 8. The mutant-insect olfactory receptor protein according to any one of Items 1 to 7, wherein the amino acid sequence A is a sequence present closer to the C-terminus of the amino acid sequence of the wild-type insect olfactory receptor protein.
Item 9. The mutant-insect olfactory receptor protein according to any one of Items 1 to 8, wherein the wild-type insect olfactory receptor protein is a wild-type olfactory receptor protein of an insect in the superorder *Endopterygota.*
Item 10. A polynucleotide comprising a coding sequence of the mutant-insect olfactory receptor protein of any one of Items 1 to 9.
Item 11. A cell comprising the polynucleotide of Item 10.
Item 12. A non-human animal comprising the cell of Item 11.
Item 13. A chemical substance detection element comprising the mutant-insect olfactory receptor protein of any one of Items 1 to 9.
Item 13A. Use of the mutant-insect olfactory receptor protein of any one of Items 1 to 9 in a chemical substance detection element.
Item 13B. Use of the mutant-insect olfactory receptor protein of any one of Items 1 to 9 in the production of a chemical substance detection element.
Item 14. A chemical substance detection sensor comprising a lipid bilayer, a cell, or a non-human animal containing the cell, the lipid bilayer and the cell each containing the chemical substance detection element of Item 13.
Item 14A. Use of a lipid bilayer, a cell, or a non-human animal containing the cell in a chemical substance detection sensor, the lipid bilayer and the cell each containing the mutant-insect olfactory receptor protein of any one of Items 1 to 9.
Item 14B. Use of a lipid bilayer, a cell, or a non-human animal containing the cell in the production of a chemical substance detection sensor, the lipid bilayer and the cell each containing the mutant-insect olfactory receptor protein of any one of Items 1 to 9.
Item 15. A method for detecting a chemical substance, comprising brining the mutant-insect olfactory receptor protein of any one of Items 1 to 9, the chemical substance detection element of Item 13, or the chemical substance detection sensor of Item 14 into contact with a chemical substance.

### Advantageous Effects of Invention

The present disclosure provides a technique of increasing the response activity of an olfactory receptor to a chemical substance.

### Brief Description of Drawings

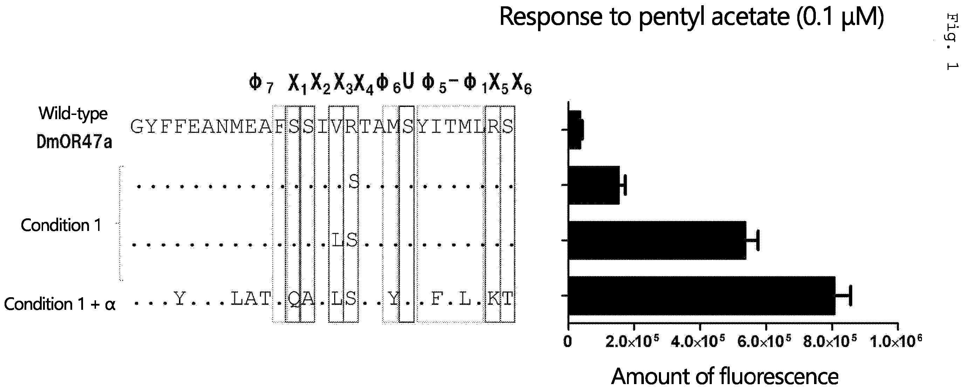

Fig. 1 shows the results of measuring chemical substance (pentyl acetate) response activity of *Drosophila* olfactory receptor 47a and mutants thereof (Test Example 1). The abscissa shows the amount of fluorescence (= activity intensity), whereas the ordinate shows the olfactory receptors and their amino acid sequences. The olfactory receptors are, from top to bottom, a wild type (Comparative Example 1-1), a mutant (RX₄S, Example 1-1), a mutant (VX₃L-RX₄S, Example 1-2), and a mutant (VX₃L-RX₄S + alpha, Example 1-3). In the amino acid sequences, "." indicates that the amino acid is identical to that of the wild type (with no mutation introduced). The concentration of the test substance in the culture medium is shown at the top of the graph.

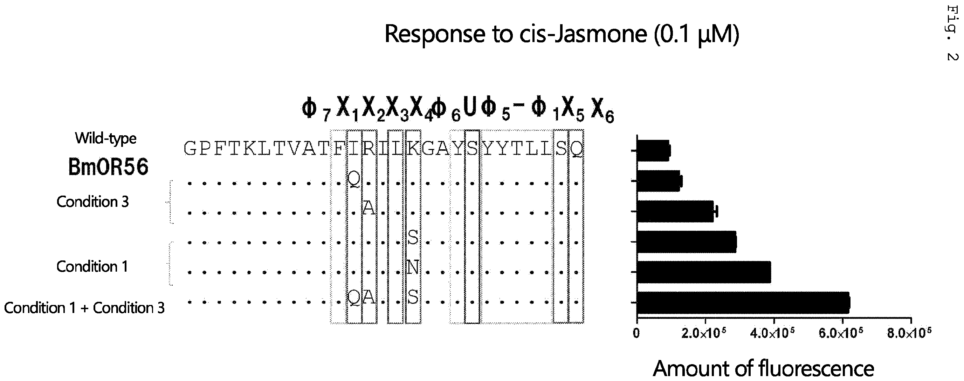

Fig. 2 shows the results of measuring chemical substance (cis-Jasmone) response activity of *Bombyx* olfactory receptor 56 and mutants thereof (Test Example 2). The abscissa shows the amount of fluorescence (= activity intensity), whereas the ordinate shows the olfactory receptors and their amino acid sequences. The olfactory receptors are, from top to bottom, a wild type (Comparative Example 2-1), a mutant (IX₁Q, Example 2-1), a mutant (RX₂A, Example 2-2), a mutant (KX₄S, Example 2-3), a mutant (KX₄N, Example 2-4), and a mutant (IX₁Q-RX₂A-KX₄S, Example 2-5). In the amino acid sequences, "." indicates that the amino acid is identical to that of the wild type (with no mutation introduced). The concentration of the test substance in the culture medium is shown at the top of the graph.

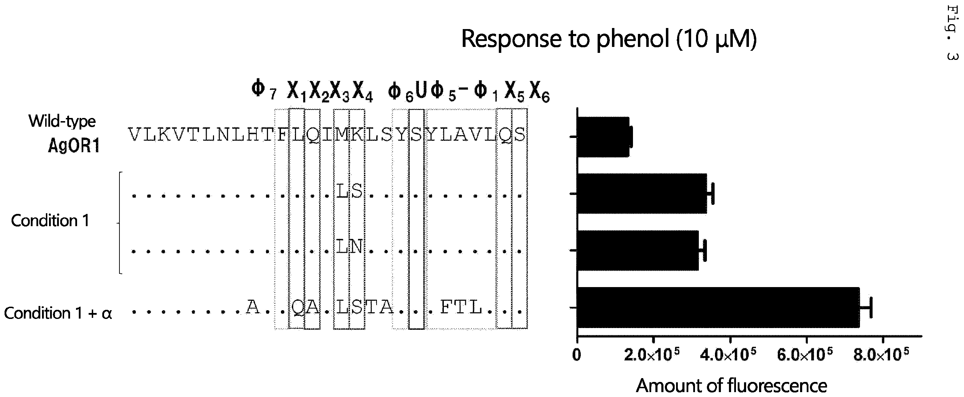

Fig. 3 shows the results of measuring chemical substance (phenol) response activity of *Anopheles* olfactory receptor 1 and mutants thereof (Test Example 3). The abscissa shows the amount of fluorescence (= activity intensity), whereas the ordinate shows the olfactory receptors and their amino acid sequences. The olfactory receptors are, from top to bottom, a wild type (Comparative Example 3-1), a mutant (MX₃L-KX₄S, Example 3-1), a mutant (MX₃L-KX₄N, Example 3-2), and a mutant (MX₃L-KX₄S + alpha, Example 3-3). In the amino acid sequences, "." indicates that the amino acid is identical to that of the wild type (with no mutation introduced). The concentration of the test substance in the culture medium is shown at the top of the graph.

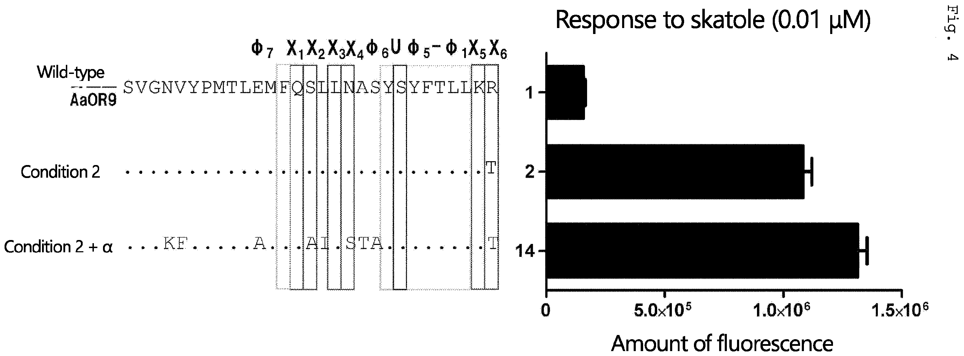

Fig. 4 shows the results of measuring chemical substance (skatole) response activity of *Aedes* olfactory receptor 9 and mutants thereof (Test Example 4). The abscissa shows the amount of fluorescence (= activity intensity), whereas the ordinate shows the olfactory receptors and their amino acid sequences. The olfactory receptors are, from top to bottom, a wild type (Comparative Example 4-1), a mutant (RX₆T, Example 4-1), and a mutant (RX₆T + alpha, Example 4-2). In the amino acid sequences, "." indicates that the amino acid is identical to that of the wild type (with no mutation introduced). The concentration of the test substance in the culture medium is shown at the top of the graph.

### Description of Embodiments

### 1. Definition

In the present specification, the terms "comprising," "containing," and "including" include the concepts of comprising, containing, consisting essentially of, and consisting of.

The "identity" of amino acid sequences refers to the degree to which two or more contrastable amino acid sequences match each other. Thus, the higher the degree of match between two amino acid sequences, the higher the identity or similarity of those sequences. The level of amino acid sequence identity is determined, for example, by using FASTA, which is a tool for sequence analysis, with default parameters. Alternatively, the level of amino acid sequence identity can be determined by using the BLAST algorithm by Karlin and Altschul (Karlin S, Altschul SF. Methods for assessing the statistical significance of molecular sequence features by using general scorings schemes, Proc Natl Acad Sci USA. 87: 2264-2268 (1990), Karlin S, Altschul SF. Applications and statistics for multiple high-scoring segments in molecular sequences, Proc Natl Acad Sci USA. 90: 5873-7 (1993)). A program called "BLASTX," based on this BLAST algorithm, has been developed. The specific techniques of these analysis methods are known and can be found on the website of the National Center of Biotechnology Information (NCBI) (http://www.ncbi.nlm.nih.gov/). The "identity" of base sequences is also defined in the same manner as above.

In the present specification, "conservative substitution" means the substitution of an amino acid residue with an amino acid residue having a similar side chain. For example, the substitution between amino acid residues having a basic side chain such as lysine, arginine, or histidine is considered to be a conservative substitution. The following substitutions between other amino acid residues are also considered to be a conservative substitution: the substitution between amino acid residues having an acidic side chain such as aspartic acid or glutamic acid; the substitution between amino acid residues having an uncharged polar side chain such as glycine, asparagine, glutamine, serine, threonine, tyrosine, or cysteine; the substitution between amino acid residues having a nonpolar side chain such as alanine, valine, leucine, isoleucine, proline, phenylalanine, methionine, or tryptophan; the substitution between amino acid residues having a β-branched side chain such as threonine, valine, or isoleucine; and the substitution between amino acid residues having an aromatic side chain such as tyrosine, phenylalanine, tryptophan, or histidine.

In the present specification, nucleotides such as DNA and RNA may have known chemical modification as illustrated below. To prevent the degradation by hydrolases such as nucleases, the phosphate residue (phosphate) of each nucleotide can be substituted with a chemically modified phosphate residue, such as phosphorothioate (PS), methylphosphonate, or phosphorodithionate. The hydroxyl group at position 2 of the ribose of each ribonucleotide may also be substituted with -OR (R indicates, for example, CH₃(2'-O-Me), CH₂CH₂OCH₃(2'-O-MOE), CH₂CH₂NHC(NH)NH₂, CH₂CONHCH₃, or CH₂CH₂CN). Additionally, the base moiety (pyrimidine, purine) may be chemically modified, by, for example, introduction of a methyl group or a cationic functional group into position 5 of the pyrimidine base, or substitution of the carbonyl group at position 2 with thiocarbonyl. Additionally, the polynucleotide of the present invention also includes, but is not limited to, those formed by modifying the phosphate moiety or the hydroxyl portion, for example, by biotin, an amino group, a lower alkyl amine group, or an acetyl group. The polynucleotide for use can also be, for example, preferably BNA (LNA), which is prepared by crosslinking the 2' oxygen and the 4' carbon in the ribose moiety of a nucleotide to fix the ribose moiety in N-conformation.

The olfactory receptor protein is a membrane protein with a seven-transmembrane structure, which is sequentially organized from the following elements linked in order from the amino terminus ("N-terminus" below) to the carboxyl terminus ("C-terminus" below): the N-terminal domain (NT), the first transmembrane domain (TM1), the first extracellular loop (EC1), the second transmembrane domain (TM2), the first intracellular loop (IC1), the third transmembrane domain (TM3), the second extracellular loop (EC2), the fourth transmembrane domain (TM4), the second intracellular loop (IC2), the fifth transmembrane domain (TM5), the third extracellular loop (EC3), the sixth transmembrane domain (TM6), the third intracellular loop (IC3), the seventh transmembrane domain (TM7), and the C-terminal domain (CT). In the present disclosure, each domain is determined by structure prediction using TMPred with conditions set to default (K. Hofmann, W. Stoffel, TMbase - a database of membrane spanning proteins segments, Biol. Chem. Hoppe-Seyler, 374 (1993), p. 166, https://embnet.vital-it.ch/software/TMPRED form.html).

In the present specification, the mutation of an amino acid is specifically deletion, substitution, insertion, or addition of an amino acid. The olfactory receptor protein may be deficient with an amino acid that has little effect on chemical substance response activity, such as an olfactory receptor peptide deficient with some amino acids other than the transmembrane domain.

In the present specification, OR stands for an olfactory receptor or odorant receptor, Orco stands for an olfactory receptor co-receptor or odorant receptor co-receptor, Dm stands for *Drosophila melanogaster,* Bm stands for *Bombyx mori,* Ag stands for *Anopheles gambiae,* and Aa stands for *Aedes aegypti.*

In the present specification, the phrase "chemical substance response activity" refers to the properties that an olfactory receptor recognizes a chemical substance, and then an olfactory receptor complex formed from the olfactory receptor and an olfactory receptor co-receptor is activated to show ion channel activity. The chemical substance response activity of an olfactory receptor can be measured with the ion channel activity of an olfactory receptor complex formed from the olfactory receptor that has come into contact with a chemical substance and an olfactory receptor co-receptor as an index. For example, cells expressing (a) an olfactory receptor, (b) an olfactory receptor co-receptor, or (c) a protein that develops color or emits light due to ions (e.g., calcium ions) flown into the cells upon response of an olfactory receptor are brought into contact with a chemical substance, and the amount of luminescence of the cells is measured. As the amount of luminescence measured is higher, the chemical substance response activity of the olfactory receptor to the chemical substance is determined to be higher. Specific examples of the measurement of chemical substance response activity are disclosed in Test Example 1-2 and Test Example 1-3, described later.

The chemical substances (e.g., odorants) for which olfactory receptors have response activity are known, or can be determined according to a known screening method. For example, the chemical substance on which DmOR47a has response activity contains pentyl acetate. The chemical substance on which BmOR56 has response activity contains cis-Jasmone. The chemical substance on which AgOR1 has response activity contains phenol. The chemical substance on which AaOR9 has response activity contains skatole. The chemical substance on which AgOR2 has response activity contains indole.

### 2. Olfactory Receptor Protein

In an embodiment, the present disclosure relates to a mutant-insect olfactory receptor protein comprising amino acid sequence B composed of amino acid sequence A containing a mutation, wherein the amino acid sequence A is in a corresponding wild-type insect olfactory receptor protein and represented by formula (1): φ₇X₁X₂Z₁X₃X₄Z₂Z₃φ₆Uφ₅φ₄φ₃φ₂φ₁X₅X₆, satisfying at least one condition selected from the group consisting of conditions 1 to 3 (which may be referred to as "the olfactory receptor protein of the present disclosure" in the present specification). The following describes the mutant-insect olfactory receptor protein.

The olfactory receptor protein of the present disclosure is a mutant-insect olfactory receptor protein composed of a wild-type insect olfactory receptor protein into which a mutation of an amino acid is introduced.

The wild-type insect olfactory receptor protein can be any olfactory receptor that is derived from an insect, i.e., an olfactory receptor intrinsically expressed (inherently possessed) by an insect, and that contains the amino acid sequence A represented by formula (1): φ₇X₁X₂Z₁X₃X₄Z₂Z₃φ₆Uφ₅φ₄φ₃φ₂φ₁X₅X₆.

In formula (1), X₁ to X₆ and Z₁ to Z₃ indicate amino acids derived from an amino acid sequence of a wild-type insect olfactory receptor. For example, if the sequence corresponding to the amino acid sequence A is FSSIVRTAMSYITMLRS (a partial sequence in the amino acid sequence of SEQ ID NO: 1) in a wild-type insect olfactory receptor, the underlined amino acids correspond to X₁, X₂, Z₁, X₃, X₄, Z₂, Z₃, X₅, and X₆ from the left.

In formula (1), φ₁ represents a hydrophobic amino acid. Examples of hydrophobic amino acids include alanine, valine, leucine, isoleucine, methionine, proline, phenylalanine, and tryptophan. Of these, hydrophobic amino acids are preferably leucine, methionine, isoleucine, valine, alanine, phenylalanine, and the like, more preferably leucine, methionine, isoleucine, valine, and the like, still more preferably leucine, methionine, and the like, and yet more preferably leucine.

In formula (1), φ₂ to φ₇ each independently represent an uncharged polar amino acid or a hydrophobic amino acid. Examples of uncharged polar amino acids include glycine, threonine, serine, asparagine, glutamine, tyrosine, and cysteine. Examples of hydrophobic amino acids include alanine, valine, leucine, isoleucine, methionine, proline, phenylalanine, and tryptophan.

φ₂ is preferably a hydrophobic amino acid, more preferably leucine, phenylalanine, isoleucine, valine, methionine, or the like, and still more preferably leucine.

φ₃ is preferably threonine, alanine, methionine, leucine, or the like, and more preferably threonine.

φ₄ is preferably phenylalanine, leucine, tyrosine, isoleucine, valine, or the like, and more preferably phenylalanine, leucine, tyrosine, or the like.

φ₅ is preferably tyrosine, phenylalanine, isoleucine, cysteine, methionine, or the like, and more preferably tyrosine.

φ₆ is preferably tyrosine, phenylalanine, glycine, tryptophan, valine, leucine, isoleucine, alanine, serine, methionine, or the like, more preferably tyrosine, phenylalanine, glycine, tryptophan, or the like, and still more preferably tyrosine, phenylalanine, or the like.

φ₇ is preferably phenylalanine, tyrosine, leucine, methionine, asparagine, serine, valine, cysteine, alanine, glycine, threonine, or the like, more preferably phenylalanine, tyrosine, leucine, or the like, and still more preferably phenylalanine, tyrosine, or the like.

In formula (1), U represents an uncharged polar amino acid. Examples of uncharged polar amino acids include glycine, threonine, serine, asparagine, glutamine, tyrosine, and cysteine. Of these, uncharged polar amino acids are preferably serine, threonine, glutamine, asparagine, glycine, and the like, more preferably serine, threonine, glutamine, and the like, and still more preferably serine, threonine, and the like.

The amino acid sequence A can be at any position in a wild-type insect olfactory receptor protein. From its sequence characteristics, the amino acid sequence A is typically a sequence present closer to the C-terminus of the amino acid sequence of a wild-type insect olfactory receptor protein. "Being closer to the C-terminus" means, for example, being present in a region from the amino acid at the C-terminus of a wild-type insect olfactory receptor protein to, for example, the 70th, preferably 60th, more preferably 50th, still more preferably 40th, and yet more preferably 35th amino acid in the wild-type insect olfactory receptor protein.

The insect that can be the origin of the wild-type insect olfactory receptor protein is preferably an insect in the superorder *Endopterygota,* more preferably an insect in the order *Diptera* such as the family *Culicidae* and the family *Drosophilidae,* an insect in the order *Lepidoptera* such as the family *Bombycidae,* and an insect in the order *Hymenoptera* such as the family *Apidae;* more preferably an insect in the order *Dipterainsect,* such as the family *Culicidae,* and the family *Drosophilidae;* and still more preferably an insect in the family *Culicidae.* Examples of insects in the family *Culicidae* include *Anopheles gambiae, Aedes aegypti,* and *Culex quinquefasciatus.* Examples of insects in the family *Drosophilidae* include *Drosophila melanogaster, Drosophila pseudoobscura,* and *Drosophila virillis.* Examples of insects in the family *Bombycidae* include *Bombyx mori, Bombyx mandarina,* and *Trilocha varians.* Examples of insects in the family *Apidae* include *Apis mellifera,* Apis florea, *Apis dorsata,* and *Bombus terrestris.*

Specific examples of the wild-type insect olfactory receptor protein include DmOR47a (amino acid sequence: SEQ ID NO: 1), BmOR56 (amino acid sequence: SEQ ID NO: 2), AgOR1 (amino acid sequence: SEQ ID NO: 3), AaOR9 (amino acid sequence: SEQ ID NO: 4), AgOR28 (amino acid sequence: SEQ ID NO: 65), AgOR47 (amino acid sequence: SEQ ID NO: 66), AgOR11 (amino acid sequence: SEQ ID NO: 67), AgOR27 (amino acid sequence: SEQ ID NO: 68), AaOR5 (amino acid sequence: SEQ ID NO: 69), AaOR31 (amino acid sequence: SEQ ID NO: 70), AaOR72 (amino acid sequence: SEQ ID NO: 71), AaOR110 (amino acid sequence: SEQ ID NO: 72), AgOR2 (amino acid sequence: SEQ ID NO: 73), AgOR10 (amino acid sequence: SEQ ID NO: 74), AaOR15 (amino acid sequence: SEQ ID NO: 75), and the like.

The olfactory receptor protein of the present disclosure comprises amino acid sequence B formed of amino acid sequence A containing a mutation. The mutation is preferably substitution.

The olfactory receptor protein of the present disclosure satisfies at least one condition selected from the group consisting of conditions 1 to 3, with respect to the amino acid sequence B.

The condition 1 is that in the amino acid sequence B, X₃ and/or X₄ is substituted, and X₃ is a branched-chain amino acid and/or X₄ is an uncharged polar amino acid.

In the condition 1, "X₃ and/or X₄ is substituted" indicates that X₃ and/or X₄ in the amino acid sequence B is substituted relative to (i.e., is an amino acid different from) X₃ and/or X₄ in the amino acid sequence A.

In the condition 1, examples of the branched-chain amino acid represented by X₃ in the amino acid sequence B include valine, leucine, isoleucine, and the like, with isoleucine, leucine, and the like being preferable.

In the condition 1, examples of the uncharged polar amino acid represented by X₄ in the amino acid sequence B include glycine, threonine, serine, asparagine, glutamine, tyrosine, cysteine, and the like, with serine, asparagine, and the like being preferable.

In the condition 1, it is preferred that X₃ in the amino acid sequence B is a branched-chain amino acid and that X₄ in the amino acid sequence B is an uncharged polar amino acid.

The condition 2 is that if X₅ and X₆ in the amino acid sequence A are both a positively charged polar amino acid, X₆ in the amino acid sequence B is an amino acid other than a positively charged polar amino acid. That is, the condition 2 is a condition limited to the case in which X₅ and X₆ in the amino acid sequence A are both a positively charged polar amino acid.

In the condition 2, examples of the positively charged polar amino acid represented by X₅ or X₆ in the amino acid sequence A include lysine, arginine, histidine, and the like, with lysine, arginine, and the like being preferable.

In the condition 2, the amino acid represented by X₆ in the amino acid sequence B is not particularly limited as long as it is an amino acid other than a positively charged polar amino acid. The amino acid is preferably, for example, an uncharged polar amino acid. Examples of uncharged polar amino acids include glycine, threonine, serine, asparagine, glutamine, tyrosine, cysteine, and the like, with serine, threonine, and the like being particularly preferable.

The condition 3 is that if either X₁ or X₂ or both in the amino acid sequence A are a positively charged polar amino acid, in the amino acid sequence B, X₁ and/or X₂ is substituted, and X₁ is an uncharged polar amino acid and/or X₂ is a hydrophobic amino acid. That is, the condition 3 is a condition limited to the case in which either X₁ or X₂ or both in the amino acid sequence A are a positively charged polar amino acid.

In the condition 3, it is preferred that at least X₂ of X₁ and X₂ in the amino acid sequence A is a positively charged polar amino acid, and it is more preferred that only X₂ in the amino acid sequence A is a positively charged polar amino acid.

In the condition 3, examples of the positively charged polar amino acid represented by X₁ or X₂ in the amino acid sequence A include lysine, arginine, histidine, and the like, with lysine, arginine, and the like being preferable, and arginine being more preferable.

In the condition 3, "X₁ and/or X₂ is substituted" indicates that X₁ and/or X₂ in the amino acid sequence B is substituted relative to (i.e., is an amino acid different from) X₁ and/or X₂ in the amino acid sequence A.

In the condition 3, examples of the uncharged polar amino acid represented by X₁ in the amino acid sequence B include glycine, threonine, serine, asparagine, glutamine, tyrosine, cysteine, and the like, with serine, glutamine, and the like being preferable.

In the condition 3, examples of the hydrophobic amino acid represented by X₂ in the amino acid sequence B include alanine, valine, leucine, isoleucine, methionine, proline, phenylalanine, tryptophan, and the like, with alanine, valine, leucine, isoleucine, and the like being preferable, and alanine being more preferable.

In the condition 3, it is preferred that X₁ in the amino acid sequence B is an uncharged polar amino acid and that X₂ in the amino acid sequence B is a hydrophobic amino acid.

In one embodiment of the present disclosure, the olfactory receptor protein of the present disclosure preferably satisfies at least one condition selected from the group consisting of conditions 1A to 3A, with respect to the amino acid sequence B.

The condition 1A is that in the amino acid sequence B, X₃ is a branched-chain amino acid and/or X₄ is an uncharged polar amino acid. The condition 1A is the same as the condition 1, except that X₃ and/or X₄ in the amino acid sequence B may or may not be substituted. That is, the condition 1A includes the case in which X₃ and/or X₄ in the amino acid sequence B is not substituted relative to the amino acid sequence A.

The condition 2A is that in the amino acid sequence B, X₆ is an amino acid other than a positively charged polar amino acid. The condition 2A is the same as the condition 2, except that X₅ and X₆ in the amino acid sequence A may or may not both be a positively charged polar amino acid. That is, the condition 2A includes the case in which X₅ and/or X₆ in the amino acid sequence B is not substituted.

The condition 3A is that in the amino acid sequence B, X₁ is an uncharged polar amino acid, and/or X₂ is a hydrophobic amino acid. The condition 3A is the same as the condition 3, except that either X₁ or X₂ or both in the amino acid sequence A may or may not be a positively charged polar amino acid. That is, the condition 3A includes the case in which X₁ and/or X₂ in the amino acid sequence B is not substituted relative to the amino acid sequence A.

The olfactory receptor protein of the present disclosure may contain amino acid mutations other than those in the conditions 1 to 3 described above, as long as the chemical substance response activity is not significantly reduced. The phrase "not significantly reduced" means, for example, that the chemical substance response activity of the olfactory receptor protein of the present disclosure containing one or more other amino acid mutations is higher than the chemical substance response activity of a corresponding wild-type insect olfactory receptor and is, for example, 50% or more, preferably 60% or more, more preferably 70% or more, still more preferably 80% or more, and yet more preferably 90% or more, of the chemical substance response activity of the olfactory receptor protein of the present disclosure that contains no other amino acid mutations taken as 100%.

The amino acid sequence of the olfactory receptor protein of the present disclosure has, for example, at least 85%, preferably at least 90%, more preferably at least 95%, and even more preferably at least 97% identity to the amino acid sequence of the mutant-insect olfactory receptor containing only the mutation(s) in at least one condition selected from the group consisting of the conditions 1 to 3.

When the olfactory receptor protein of the present disclosure contains one or more other amino acid mutations, the amino acid sequence of the olfactory receptor protein of the present disclosure is preferably an amino acid sequence in which one or multiple amino acids are mutated relative to the amino acid sequence of the mutant-insect olfactory receptor containing only the mutation(s) in at least one condition selected from the group consisting of the conditions 1 to 3. The term "multiple" means, for example, 2 to 50, preferably 2 to 30, more preferably 2 to 20, still more preferably 2 to 10, and yet more preferably 2 to 5.

Examples of the other amino acid mutations include substitution, deletion, addition, insertion, and the like, with substitution being preferable, and conservative substitution being particularly more preferably. In addition, in the case of mutations in φ₂ to φ₇ in formula (1), substitution of uncharged polar amino acids and hydrophobic amino acids is also preferred.

The chemical substance response activity of the olfactory receptor protein of the present disclosure is higher than the chemical substance response activity of a corresponding wild-type insect olfactory receptor. The chemical substance response activity of the olfactory receptor protein of the present disclosure is, for example, 1.2 times or more, 1.5 times or more, 2 times or more, 3 times or more, 4 times or more, 5 times or more, 6 times or more, 7 times or more, 8 times or more, 9 times or more, 10 times or more, 15 times or more, or 20 times or more the chemical substance response activity of a corresponding wild-type insect olfactory receptor.

The olfactory receptor protein of the present disclosure may have other amino acid sequences, such as a protein or peptide (e.g., a protein tag, a fluorescent protein, a photoprotein, or a signal sequence), added thereto, as long as the chemical substance response activity is not significantly impaired. Examples of protein tags include biotin, a His tag, a FLAG tag, a Halo tag, an MBP tag, a HA tag, a Myc tag, a V5 tag, a PA tag, and the like.

The olfactory receptor protein of the present disclosure may be chemically modified, as long as the chemical substance response activity is not significantly impaired.

The olfactory receptor protein of the present disclosure may have a carboxyl group (-COOH), carboxylate (-COO⁻), amide (-CONH₂), or ester (-COOR) at the C-terminus.

"R" in the ester is, for example, a C₁₋₆ alkyl group, such as methyl, ethyl, n-propyl, isopropyl, or n-butyl; a C₃₋₈ cycloalkyl group, such as cyclopentyl or cyclohexyl; a C₆₋₁₂ aryl group, such as phenyl or α-naphthyl; a phenyl-C₁₋₂ alkyl group, such as benzyl or phenethyl; a C₇₋₁₄ aralkyl group including an α-naphthyl-C₁₋₂ alkyl group, such as α-naphthylmethyl; a pivaloyloxymethyl group; or the like.

The olfactory receptor protein of the present disclosure may have an amidated or esterified carboxyl group (or carboxylate), which is not the carboxyl group at the C-terminus. As the ester in this case, for example, the above-mentioned C-terminal ester or the like is used.

Furthermore, the olfactory receptor protein of the present disclosure also includes those in which the amino group of the N-terminal amino acid residue is protected by a protecting group (e.g., a C₁₋₆ acyl group such as C₁₋₆ alkanoyl, such as a formyl group or an acetyl group); those in which the N-terminal glutamine residue that can be produced by cleavage in vivo is converted to pyroglutamic acid; those in which a substituent (e.g., -OH, -SH, an amino group, an imidazole group, an indole group, or a guanidino group) on an amino acid side chain in the molecule is protected by an appropriate protecting group (e.g., a C₁₋₆ acyl group such as C₁₋₆ alkanoyl, such as a formyl group or an acetyl group); conjugated proteins such as those called glycoproteins having a sugar chain bound thereto; and the like.

The olfactory receptor protein of the present disclosure may be in the form of a salt with an acid or a base. The salt is not particularly limited, and acid salts and basic salts can both be employed. Examples of acid salts include inorganic acid salts, such as hydrochloride, hydrobromide, sulfate, nitrate, and phosphate; organic acid salts, such as acetate, propionate, tartrate, fumarate, maleate, malate, citrate, methanesulfonate, and p-toluenesulfonate; amino acid salts, such as aspartate and glutamate; and the like. Examples of basic salts include alkali metal salts, such as sodium salt and potassium salt; alkaline earth metal salts, such as calcium salt and magnesium salt; and the like.

The olfactory receptor protein of the present disclosure may be in the form of a solvate. The solvent is not particularly limited, and examples include water, ethanol, glycerol, acetic acid, and the like.

The olfactory receptor protein of the present disclosure can be easily produced according to a known genetic engineering method. For example, the olfactory receptor protein of the present disclosure can be produced using PCR, restriction enzyme cleavage, a DNA ligation technique, an in vitro transcription/translation technique, a recombinant protein production technique, etc.

### 3. Polynucleotide, Cell, and Non-human Animal

In one embodiment, the present disclosure relates to a polynucleotide comprising a coding sequence of the olfactory receptor protein of the present disclosure (which may be referred to as "the polynucleotide of the present disclosure" in the present specification), a cell comprising the polynucleotide of the present disclosure (which may be referred to as "the cell of the present disclosure" in the present specification), and a non-human animal comprising the cell of the present disclosure (which may be referred to as "the non-human animal of the present disclosure" in the present specification). These are described below.

The coding sequence of the olfactory receptor protein of the present disclosure is not particularly limited as long as it is a polynucleotide comprising a base sequence encoding the olfactory receptor protein of the present disclosure.

In one embodiment, the polynucleotide of the present disclosure include an expression cassette for the olfactory receptor protein of the present disclosure.

The expression cassette for the olfactory receptor protein of the present disclosure is not particularly limited as long as it is a polynucleotide capable of expressing the olfactory receptor protein of the present disclosure in a cell. Typical examples of the expression cassette for the olfactory receptor protein of the present disclosure include a polynucleotide containing a promoter and the coding sequence of the olfactory receptor protein of the present disclosure placed under the control of the promoter.

The promoter contained in the expression cassette for the olfactory receptor protein of the present disclosure is not particularly limited, and can be appropriately selected depending on the target cell. For example, various pol II promoters can be used. Examples of pol II promoters include, but are not limited to, a CMV promoter, an EF1 promoter, an SV40 promoter, an MSCV promoter, and the like. Other examples of the promoter include a tryptophan promoter such as trc or tac, a lac promoter, a T7 promoter, a T5 promoter, a T3 promoter, an SP6 promoter, an arabinose-inducible promoter, a cold-shock promoter, a tetracycline-inducible promoter, and the like.

The expression cassette for the olfactory receptor protein of the present disclosure may contain other elements (e.g., a multiple cloning site (MCS), a drug resistance gene, an origin of replication, an enhancer sequence, a repressor sequence, an insulator sequence, a reporter protein (e.g., fluorescent protein) coding sequence, and a drug resistance gene coding sequence), if necessary.

The polynucleotide of the present disclosure may be in the form of a vector. An appropriate vector is selected according to the intended use (cloning, expression of protein) and in consideration of the kind of host cell. Examples of a vector for *Escherichia coli* as a host include M13 phage or a modified form thereof, λ phage or a modified form thereof, pBR322 or a modified form thereof (e.g., pB325, pAT153, and pUC8), and the like; examples of a vector for a yeast as a host include pYepSec1, pMFa, pYES2, pPIC3.5K, and the like; examples of a vector for an insect cell as a host include pAc, pVL, and the like; and examples of a vector for a mammalian cell as a host include pcDNA, pCDM8, pMT2PC, and the like.

The cell of the present disclosure is not particularly limited as long as it contains the polynucleotide of the present disclosure. Examples of the cell include colon bacteria such as *Escherichia coli* K12; *Bacillus* bacteria such as *Bacillus subtilis* MI114; yeasts such as *Saccharomyces cerevisiae* AH22; insect cells, such as *Spodoptera frugiperda-derived* Sf cell line, *Trichoplusia ni*-derived High Five cell line, and olfactory neurons; animal cells such as COS7 cells; and the like. Preferred examples of animal cells include mammal-derived cultured cells, such as COS7 cells, CHO cells, HEK293 cells, HEK293FT cells, Hela cells, PC12 cells, N1E-115 cells, SH-SY5Y cells, and the like.

From the viewpoint that the cell of the present disclosure can be used as is for the chemical substance detection described later, the cell of the present disclosure preferably contains a coding sequence for an insect olfactory receptor co-receptor. The insect olfactory receptor co-receptor is a membrane protein having a seven-transmembrane structure as in olfactory receptors; however, the insect olfactory receptor co-receptor itself does not recognize odorants and functions by forming a heterocomplex with an olfactory receptor. The olfactory receptor complex, a heterocomplex composed of an olfactory receptor and an olfactory receptor co-receptor, has ion channel activity that is activated by an odorant, allowing cations such as sodium ions (Na⁺) and calcium ions (Ca²⁺) to flow into cells when activated.

From the same viewpoint, the cell of the present disclosure preferably contains a coding sequence for a protein that develops color or emits light due to ions (e.g., calcium ions) flown into the cell upon response of an olfactory receptor. Examples of the protein include aequorin, yellow cameleon (YC), GCaMP, and the like. Alternatively, the cell of the present disclosure preferably contains a calcium ion-dependent fluorescent dye (e.g., Fura-2, Fluo-3, or Fluo-4).

From the same viewpoint, the cell of the present disclosure contains the olfactory receptor protein of the present disclosure, i.e., the olfactory receptor protein of the present disclosure is expressed in the cell of the present disclosure. In this case, the olfactory receptor protein of the present disclosure, which has a seven-transmembrane structure, is positioned as a membrane protein on the cell membrane.

The non-human animal of the present disclosure is not particularly limited as long as it comprises the cell of the present disclosure. Although there is no particular limitation, the non-human animal is preferably an insect, from the viewpoint of being suitable for use in the chemical substance detection described later. The insect is as described in the "2. Olfactory Receptor Protein" section above. From the same viewpoint, it is also preferred that the cells of the present disclosure in the non-human animal of the present disclosure comprise olfactory neurons.

### 4. Use

The olfactory receptor protein of the present disclosure has the activity of allowing cations (sodium ions (Na⁺), calcium ions (Ca²⁺), or the like) to flow into cells in response to a chemical substance. The chemical substance can be detected by detecting the cations or by detecting behavioral changes based on olfactory neuron activation induced by the influx of the cations. Thus, the olfactory receptor protein of the present disclosure can be used as a chemical substance detection element.

In one embodiment, the present disclosure relates to a chemical substance detection element comprising the olfactory receptor protein of the present disclosure (the detection element of the present disclosure), and further relates to a chemical substance detection sensor that comprises a cell comprising the detection element of the present disclosure, a lipid bilayer (artificial cell membrane) comprising the detection element of the present disclosure, or a non-human animal comprising the cell (the detection sensor of the present disclosure).

Chemical substance detection sensors using cells, lipid bilayers, or non-human animals are already known, and as their specific configurations, the configurations described in, for example, PTL 1, PTL 2, PTL 3, etc. can be used. The chemical substance detection sensor comprising the cell or lipid bilayer of the present disclosure comprises, for example, a container holding the cell or lipid bilayer of the present disclosure, a sensor that outputs a signal upon detection of a chemical substance response (e.g., light) in the cell of the present disclosure, and a determiner that detects a chemical substance based on the signal. The chemical substance detection sensor comprising the non-human animal of the present disclosure comprises, for example, a detector (e.g., a motion sensor, a vibration sensor, or a sound sensor) that detects movement of the non-human animal of the present disclosure.

### Examples

Embodiments of the present invention are described in more detail with reference to Examples. However, the present invention is not limited to these Examples.

The wild-type olfactory receptor genes used in the following examples are as follows.

**Table 1**

| Source organism name | Wild-type gene | Base sequence: SEQ ID NO. | Amino acid sequence: SEQ ID NO. | Accession No. |
|---|---|---|---|---|
| *Drosophila Melanogaster* | DmOR47a | SEQ ID NO: 5 | SEQ ID NO: 1 | NH_078965.3 |
| *Bombyx mori* | BmOR56 | SEQ ID NO: 6 | SEQ ID NO: 2 | NH_001173146.1 |
| *Anopheles gambiae* | AgOR1 | SEQ ID NO: 7 | SEQ ID NO: 3 | XM_318674.1 |
| *Aedes aegypti* | AgOR9 | SEQ ID NO: 8 | SEQ ID NO: 4 | NH_001358324.1 |

### Test Example 1. Measurement of Chemical Substance Response Activity of DmOR47a

The chemical substance response activity of olfactory receptors (wild-type DmOR47a and DmOR47a mutants) was measured.

### Test Example 1-1. Preparation of Expression Plasmids

### Comparison Example 1-1. Preparation of Wild-type DmOR47a Expression Plasmid

Using adult *Drosophila melanogaster* RNA (produced by Takara Bio, Inc.) as a template and adding a Super Script III reverse transcriptase (produced by Invitrogen Corporation), a reverse transcription reaction was performed at 55°C for 50 minutes and then at 75°C for 15 minutes to obtain cDNA. Using 1 µL of the obtained cDNA as a template, PCR was performed using 1 µL of 10 µM forward primer DmOR47a-5' (5'-caccatggacagttttctgcaagtacagaa; SEQ NO: 9), 1 µL of 10 µM reverse primer DmOR47a-3' (5'-ttaggagaatgatctcagcattgtgatgta; SEQ ID NO: 10), and 1 µL of KOD-Plus-Neo DNA polymerase (produced by Toyobo Co., Ltd.). The PCR reaction was performed under the following conditions: (1) 94°C for 2 minutes, (2) 98°C for 10 seconds, and (3) 68°C for 1.5 minutes, in which 35 cycles of steps (2) and (3) were repeatedly performed. After the obtained PCR product was subjected to agarose gel electrophoresis, about 1.2 kb of DNA detected on the gel was collected. The recovered DNA was introduced into a pENTR/D-TOPO vector (produced by Invitrogen Corporation) to obtain a plasmid named pENTR-DmOR47a. 4 µL of pENTR-DmOR47a, 1 µL of pcDNA6.2V5-DEST, and 2 µL of LR Clonase II (produced by Invitrogen Corporation) were mixed and maintained at room temperature for 1 hour. The resulting mixture was introduced into *E. coli* and cultured to obtain an expression plasmid named pcDNA6.2-DmOR47a. The expression plasmid pcDNA6.2-DmOR47a was sequenced with a DNA sequencer. The base sequence of the expression plasmid pcDNA6.2-DmOR47a was found to contain the base sequence set forth in SEQ ID NO: 5. The base sequence of SEQ ID NO: 5 encodes the amino acid sequence of SEQ ID NO: 1. Fig. 1 shows the correspondence between the amino acid sequence of formula (1): φ₇X₁X₂Z₁X₃X₄Z₂Z₃φ₆Uφ₅φ₄φ₃φ₂φ₁X₅X₆ and a portion of the amino acid sequence of SEQ ID NO: 1 corresponding to the amino acid sequence of formula (1).

### Example 1-1. Preparation of DmOR47a Mutant (RX₄S) Expression Plasmid

A plasmid (pcDNA6.2) containing a sequence (base sequence: SEQ ID NO: 12) encoding a DmOR47a mutant (RX₄S) comprising the amino acid sequence of wild-type DmOR47a in which the 372nd amino acid residue arginine (X₄ in formula (1)) was mutated to serine (amino acid sequence: SEQ ID NO: 11) was prepared in the following manner.

PCR primers designed to convert the 372nd amino acid residue arginine (X₄ in formula (1)) in the amino acid sequence of wild-type DmOR47a to serine (primer F:
CCTTCTCATCGATTGTTAGCACGGCGATGTCCTAC (SEQ ID NO: 13) and primer R: GTAGGACATCGCCGTGCTAACAATCGATGAGAAGG (SEQ ID NO: 14)) were synthesized. Using these primers, PCR amplification was performed using pcDNA6.2-DmOR47a as a template (the reaction procedure and conditions were according to the standard method). The amplified PCR reaction solution was digested with DpnI and then transformed into *E. coli* (DH5α). The transformed *E. coli* were seeded in 25-cm square (ampicillin-supplemented) plates and cultured at 37°C to form colonies. Two colonies were selected and cultured again in LB liquid medium. The plasmids harbored in the colonies were then sequenced to obtain a DmOR47a mutant (RX₄S) expression plasmid.

### Example 1-2: Preparation of Mutant DmOR47a (VX₃L-RX₄S) Expression Plasmid

A plasmid (pcDNA6.2) containing a sequence (base sequence: SEQ ID NO: 16) encoding a DmOR47a mutant (VX₃L-RX₄S) comprising the amino acid sequence of wild-type DmOR47a in which the 371st amino acid residue valine (X₃ in formula (1)) was mutated to leucine and the 372nd amino acid residue arginine (X₄ in formula (1)) was mutated to serine (amino acid sequence: SEQ ID NO: 15) was prepared. Specifically, the plasmid was prepared in the same manner as in Example 1-1 except that primer F: CCTTCTCATCGATTCTTAGCACGGCGATGTCC (SEQ ID NO: 17) and primer R: GGACATCGCCGTGCTAAGAATCGATGAGAAGG (SEQ ID NO: 18) were used as PCR primers and the DmOR47a (RX₄S) mutant expression plasmid (Example 1-1) was used as a template for PCR.

### Example 1-3: Preparation of DmOR47a Mutant (VX₃L-RX₄S + alpha) Expression Plasmid

A plasmid (pcDNA6.2) containing a sequence (base sequence: SEQ ID NO: 20) encoding a DmOR47a mutant (VX₃L-RX₄S + alpha) (amino acid sequence: SEQ ID NO: 19), which further contained multiple mutations in the DmOR47a mutant (VX₃L-RX₄S), was prepared. Fig. 1 shows the mutation sites. Specifically, using the DmOR47a (VX₃L-RX₄S) mutant expression plasmid (Example 1-2) as a PCR template, mutations were sequentially introduced using each primer set for mutagenesis.

### Reference Example 1-1: Preparation of Chimeric Orco Expression Plasmid

A plasmid (pcDNA3.1) containing a sequence (base sequence: SEQ ID NO: 22) encoding a fusion protein Dm(NT-TM4) AmOrco (amino acid sequence: SEQ ID NO:21), which is a fusion protein of a part of *Drosophila* co-receptor and a part of *Apis* co-receptor, was prepared in accordance with a previous report (JP2018-50556A). In Dm(NT-TM4) AmOrco, the amino acid sequence from the N-terminal region (NT) to the four-transmembrane domain (TM4) (amino acid sequence from its N-terminal amino acid residue to the 234th amino acid residue) is derived from a *Drosophila* co-receptor (Dm(NT-TM4)), whereas the amino acid sequence from the second intracellular loop to the C-terminal region is derived from an *Apis* co-receptor (Am(IC2-CT)).

### Test Example 1-2. Introduction of Expression Plasmid into Cells

HEK293FT cells (purchased from Invitrogen Corporation) were seeded in 10-cm petri dishes at 3 × 10⁶ cells/petri dish and cultured in DMEM medium containing 10% FBS (produced by Nacalai Tesque, Inc.) at 37°C and 5% CO₂ for about 24 hours. 1.5 µg of any one of the olfactory receptor expression plasmids prepared, 3.0 µg of olfactory receptor co-receptor expression plasmid, and 8 µg of GFP-aequorin (GAP) expression plasmid were mixed with 12.5 µL of a Plus reagent and 31.25 µL of Lipofectamine LTX (produced by Invitrogen Corporation) and maintained for 10 minutes. This mixture was then transfected into the above cells. Four hours after the start of transfection, the cells were seeded in 96-well plates at 9 × 10⁴ cells/well and cultured in DMEM medium (produced by Nacalai Tesque, Inc.) containing 10% FBS at 37°C and 5% CO₂ for about 24 hours. A transformed cell in which the olfactory receptor expression plasmid, olfactory receptor co-receptor expression plasmid, and GAP expression plasmid were transiently introduced was thereby obtained.

### Test Example 1-3. Activity Measurement

When bound to calcium ions, GFP-aequorin (GAP) is activated and emits green fluorescence in the presence of a substrate, such as coelenterazine. Accordingly, since an increase in intracellular calcium ion concentration in GAP-expressing cells directly appears as an increase in fluorescence, whether the olfactory receptor complex functions as an ion channel upon addition of a test substance can be determined from the change in the amount of fluorescence.

The culture medium of the above transformed cells was removed and replaced with Assay buffer (Hanks-HEPES (20 mM pH 7.4) containing 0.5 µM Coelenterazine-h (produced by Promega Corporation) and 0.3% BSA), and further allowed to stand at room temperature for 4 hours. Subsequently, the test substance corresponding to the olfactory receptor was added to the cell culture medium using a FlexStation 3 (produced by Molecular Devices, LLC), and the amount of fluorescence of the cells was measured.

In this test example, pentyl acetate, a chemical substance recognized by DmOR47a, was used as a test substance.

Fig. 1 shows the results. The DmOR47a mutants obtained in Examples 1-1 to 1-3 showed higher chemical substance response activity than wild-type DmOR47a.

### Test Example 2: Measurement of Chemical Substance Response

### Activity of BmOR56

The chemical substance response activity of olfactory receptors (wild-type BmOR56 and BmOR56 mutants) was measured.

### Test Example 2-1: Preparation of Expression Plasmid

### Comparison Example 2-1. Preparation of Wild-type BmOR56 Expression Plasmid

Double-stranded DNA comprising the base sequence of SEQ ID NO: 6 on one of the DNA strands was synthesized. Using 100 ng of this double-stranded DNA as a template, PCR was performed using 1 µL of a 10 µM forward primer BmOR56-5' (5'-tggaattctgcagatcaccatgaagctcctggagaagctag; SEQ ID NO: 23), 1 µL of a 10 µM reverse primer BmOR56-3' (5'-gccactgtgctggattcatgttttattcatttgcgactgac; SEQ ID NO: 24), and 1 µL of KOD-Plus-Neo DNA polymerase (produced by Toyobo Co., Ltd.). The PCR reaction was performed under the following conditions: (1) 94°C for 2 minutes, (2) 98°C for 10 seconds, (3) 63°C for 30 seconds, and (4) 68°C for 1.5 minutes, in which 35 cycles of steps (2) to (4) were repeatedly performed. The obtained PCR product was linked to EcoRV-digested pcDNA6.2 (produced by Invitrogen Corporation) using an In-Fusion HD Cloning Kit (produced by Takara Bio, Inc.). The linked DNA was introduced into *E. coli* and cultured to obtain an expression plasmid named pcDNA6.2-BmOR56. The expression plasmid pcDNA6.2-BmOR56 was sequenced with a DNA sequencer. The base sequence of the expression plasmid pcDNA6.2-BmOR56 was found to contain the base sequence of SEQ ID NO: 6. The base sequence of SEQ ID NO: 6 encodes the amino acid sequence of SEQ ID NO: 2. Fig. 2 shows the correspondence between the amino acid sequence of formula (1): φ₇X₁X₂Z₁X₃X₄Z₂Z₃φ₆Uφ₅φ₄φ₃φ₂φ₁X₅X₆ and a portion of the amino acid sequence of SEQ ID NO: 2 corresponding to the amino acid sequence of formula (1).

### Example 2-1. Preparation of BmOR56 Mutant (IX₁Q) Expression Plasmid

Plasmid (pcDNA6.2) containing a sequence (base sequence: SEQ ID NO: 26) encoding a BmOR56 mutant (IX₂Q) comprising the amino acid sequence of wild-type BmOR56 in which the 380th amino acid residue isoleucine (X₁ in formula (1)) was mutated to glutamine (amino acid sequence: SEQ ID NO: 25) was prepared in the following manner.

CR primers designed to convert the 380th amino acid residue isoleucine (X₁ in formula (1)) in the amino acid sequence of wild-type BmOR56 to glutamine (Primer F:
CGGTCGCCACTTTTCAGAGGATCCTCAAAGGAG (SEQ ID NO: 27) and Primer R: CTCCTTTGAGGATCCTCTGAAAAGTGGCGACCG (SEQ ID NO: 28)) were synthesized. Mutagenesis was performed in the same manner as in the above Example by PCR using pcDNA6.2-BmOR56 as a template and using these primers, thus obtaining a BmOR56 mutant (IX₁Q) expression plasmid.

### Example 2-2. Preparation of BmOR56 (RX₂A) Mutant Expression Plasmid

A plasmid (pcDNA6.2) containing a sequence (base sequence: SEQ ID NO: 30) encoding a BmOR56 (RX₂A) mutant comprising the amino acid sequence of wild-type BmOR56 in which the 381st amino acid residue isoleucine (X₂ in formula (1)) was mutated to alanine (amino acid sequence: SEQ ID NO: 29) was prepared. Specifically, the plasmid was prepared in the same manner as in Example 2-1 except that primer F: GTCGCCACTTTTATCGCAATCCTCAAAGGAGCC (SEQ ID NO: 31) and Primer R: GGCTCCTTTGAGGATTGCGATAAAAGTGGCGAC (SEQ ID NO: 32) were used as PCR primers.

### Example 2-3: Preparation of BmOR56 (KX₄S) Mutant Expression Plasmid

A plasmid (pcDNA6.2) containing a sequence (base sequence: SEQ ID NO: 34) encoding a BmOR56 mutant (KX₄S) comprising the amino acid sequence of wild-type BmOR56 in which the 384th amino acid residue lysine (X₄ in formula (1)) was mutated to serine (amino acid sequence: SEQ ID NO: 33) was prepared. Specifically, the plasmid was prepared in the same manner as in Example 2-1 except that primer F:
CACTTTTATCAGGATCCTCAGCGGAGCCTATAGTTACTAC (SEQ ID NO: 35) and primer R: GTAGTAACTATAGGCTCCGCTGAGGATCCTGATAAAAGTG (SEQ ID NO: 36) were used as PCR primers.

### Example 2-4: Preparation of BmOR56 (KX₄N) Mutant Expression Plasmid

A plasmid (pcDNA6.2) containing a sequence (base sequence: SEQ ID NO: 38) encoding a BmOR56 mutant (KX₄N) comprising the amino acid sequence of wild-type BmOR56 in which the 384th amino acid residue lysine (X4 in formula (1)) was mutated to asparagine (amino acid sequence: SEQ ID NO: 37) was prepared. Specifically, the plasmid was prepared in the same manner as in Example 2-1 except that primer F:
CACTTTTATCAGGATCCTCAACGGAGCCTATAGTTACTAC (SEQ ID NO: 39) and primer R: GTAGTAACTATAGGCTCCGTTGAGGATCCTGATAAAAGTG (SEQ ID NO: 40) were used as PCR primers.

### Example 2-5. Preparation of BmOR56 Mutant (IX₁Q-RX₂A-KX₄S) Expression Plasmid

A plasmid (pcDNA6.2) containing a sequence (base sequence: SEQ ID NO: 42) encoding a BmOR56 mutant (IX₁Q-RX₂A-KX₄S) comprising the amino acid sequence of wild-type BmOR56 in which the 380th amino acid residue isoleucine (X₁ in formula (1)), the 381st amino acid residue arginine (X₂ in formula (1)), and the 384th amino acid residue lysine (X₄ in formula (1)) were mutated to glutamine, alanine, and serine, respectively (amino acid sequence: SEQ ID NO: 41) was prepared. Specifically, the plasmid was prepared in the same manner as in the above Example by sequentially introducing mutations using each primer set for mutagenesis.

### Test Example 2-2: Introduction of Expression Plasmid into Cells and Activity Measurement

The expression plasmid was introduced into cells in the same manner as in Test Example 1-2, and the activity was measured in the same manner as in Test Example 1-3. In this test example, cis-Jasmone was used as a test substance.

Fig. 2 shows the results. The BmOR56 mutants obtained in Examples 2-1 to 2-5 were found to have higher chemical substance response activity than wild-type BmOR56.

### Test Example 3: Measurement of Chemical Substance Response Activity of AgOR1

The response activity of olfactory receptors (wild-type AgOR1 and AgOR1 mutants) to chemical substances was measured.

### Test Example 3-1. Preparation of Expression Plasmid

### Comparative Example 3-1. Preparation of Wild-type AgOR1 Expression Plasmid

Double-stranded DNA comprising the base sequence of SEQ ID NO: 7 on one of the DNA strands was synthesized. Using 100 ng of this double-stranded DNA as a template, PCR was performed using 1 µL of a 10 µM forward primer AgOR1-5' (5'-tggaattctgcagatcaccatgaagctgaacaaactgaac; SEQ ID NO: 43), 1 µL of a 10 µM reverse primer AgOR1-3' (5'-gccactgtgctggatttactctgattccatgctctg; SEQ ID NO: 44), and 1 µL of KOD-Plus-Neo DNA polymerase (produced by Toyobo Co., Ltd.). The PCR reaction was performed under the following conditions: (1) 94°C for 2 minutes, (2) 98°C for 10 seconds, (3) 63°C for 30 seconds, and (4) 68°C for 1.5 minutes, in which 35 cycles of steps (2) to (4) were repeatedly performed. The obtained PCR product was linked to EcoRV-digested pcDNA6.2 (produced by Invitrogen Corporation) using an In-Fusion HD Cloning Kit (produced by Takara Bio, Inc.). The linked DNA was introduced into *E. coli* and cultured to obtain an expression plasmid named pcDNA6.2-AgOR1. The expression plasmid pcDNA6.2-AgOR1 was sequenced with a DNA sequencer and found to contain the base sequence of SEQ ID NO: 7. The base sequence of SEQ ID NO: 7 encodes the amino acid sequence of SEQ ID NO: 3. Fig. 3 shows the correspondence between the amino acid sequence of formula (1): φ₇X₁X₂Z₁X₃X₄Z₂Z₃φ₆Uφ₅φ₄φ₃φ₂φ₁X₅X₆ and a portion of the amino acid sequence of SEQ ID NO: 3 corresponding to the amino acid sequence of formula (1).

### Example 3-1. Preparation of AgOR1 Mutant (MX₃L-KX₄S) Expression Plasmid

A plasmid (pcDNA6.2) containing a sequence (base sequence: SEQ ID NO: 46) encoding an AgOR1 mutant (MX₃L-KX₄S) comprising the amino acid sequence of wild-type AgOR1 in which the 401st amino acid residue methionine (X₃ in formula (1)) was mutated to leucine and the 402nd amino acid residue lysine (X₄ in formula (1)) was mutated to serine (amino acid sequence: SEQ ID NO: 45) was prepared in the following manner.

PCR primer F: CACATTTTTGCAGATTTTGAGCCTATCGTACTCCTATC (SEQ ID NOs: 47 and 49) and PCR primer R:
GATAGGAGTACGATAGGCTCAAAATCTGCAAAAATGTG (SEQ ID NOs: 48 and 50) designed to convert the 401st amino acid residue methionine (X₃ in formula (1)) and the 402nd amino acid residue lysine (X₄ in formula (1)) in the amino acid sequence of wild-type AgOR1 to leucine and serine, respectively were synthesized. Using pcDNA6.2-AgOR1 as a template and using these primers, PCR was performed in the same manner as in the above Example to sequentially introduce mutations, thus obtaining an AgOR1 mutant (MX₃L-KX₄S) expression plasmid.

### Example 3-2: Preparation of AgOR1 Mutant (MX₃L-KX₄N) Expression Plasmid

A plasmid (pcDNA6.2) containing a sequence (base sequence: SEQ ID NO: 52) encoding an AgOR1 mutant (MX₃L-KX₄N) comprising the amino acid sequence of wild-type AgOR1 in which the 401st amino acid residue methionine (X₃ in formula (1)) and the 402nd amino acid residue lysine (X₄ in formula (1)) were mutated to leucine and asparagine, respectively (amino acid sequence: SEQ ID NO: 51) was prepared. Specifically, PCR primer F: CACATTTTTGCAGATTTTGAACCTATCGTACTCCTATC (SEQ ID NO: 53) and primer R: GATAGGAGTACGATAGGTTCAAAATCTGCAAAAATGTG (SEQ ID NO: 54)) designed to convert the 401st amino acid residue lysine (X₄ of formula (1)) and the 402nd amino acid residue lysine (X₄ in formula (1)) to asparagine were synthesized and prepared in the same manner as in Example 3-1.

### Example 3-3. Preparation of AgOR1 Mutant (MX₃L-KX₄S + alpha) Expression Plasmid

A plasmid (pcDNA6.2) containing a sequence (base sequence: SEQ ID NO: 56) encoding an AgOR1 mutant (MX₃L-KX₄S+alpha), which further contained multiple mutations in the AgOR1 mutant (MX₃L-KX₄S) (amino acid sequence: SEQ ID NO: 55), was prepared. Fig. 3 shows the mutation sites. Specifically, using the AgOR1 mutant (MX₃L-KX₄S) expression plasmid (Example 3-1) as a PCR template, mutations were sequentially introduced using each primer set of mutagenesis.

### Test Example 3-2: Introduction of Expression Plasmid into Cells and Activity Measurement Test

The expression plasmid was introduced into cells in the same manner as in Example 1-2, and the activity was measured in the same manner as in Example 1-3. In this test example, phenol was used as a test substance.

Fig. 3 shows the results. The AgOR1 mutants obtained in Examples 3-1 to 3-3 were found to have higher chemical substance response activity than wild-type AgOR1.

### Test Example 4: Measurement of Chemical Substance Response Activity of AaOR9

The chemical substance response activity of olfactory receptors (wild-type AaOR9 and AaOR9 mutants) was measured.

### Test Example 4-1: Preparation of Expression Plasmid

### Comparative Example 4-1: Preparation of Wild-type AaOR9 Expression Plasmid

Double-stranded DNA comprising the base sequence of SEQ ID NO: 8 on one of the DNA strands was synthesized. Using 100 ng of this double-stranded DNA as template, PCR was performed using 1 µL of a 10 µM forward primer AaOR9-5' (5'-tggaattctgcagatcaccatgtccgtcgagaagatcctggc; SEQ ID NO: 57), 1 µL of a 10 µM reverse primer AaOR9-3' (5'-gccactgtgctggatttagctataaacacgtttcaaaagag; SEQ sequence No. 58), and 1 µL of KOD-Plus-Neo DNA polymerase (produced by Toyobo Co., Ltd.). A PCR reaction was performed under the following conditions: (1) 94°C for 2 minutes, (2) 98°C for 10 seconds, (3) 63°C for 30 seconds, and (4) 68°C for 1.5 minutes, in which 35 cycles of Steps (2) to (4) were repeatedly performed. The resulting PCR product was linked to EcoRV-digested pcDNA6.2 (produced by Invitrogen Corporation) using an In-Fusion HD Cloning Kit (produced by Takara Bio, Inc.). The linked DNA was introduced into *E. coli* and cultured to obtain an expression plasmid named pcDNA6.2-AaOR9. The expression plasmid pcDNA6.2-AaOR9 was sequenced with a DNA sequencer and found to contain the base sequence of SEQ ID NO: 8. The base sequence of SEQ ID NO: 8 encodes the amino acid sequence of SEQ ID NO: 4. Fig. 4 shows the correspondence between the amino acid sequence of formula (1): φ₇X₁X₂Z₁X₃X₄Z₂Z₃φ₆Uφ₅φ₄φ₃φ₂φ₁X₅X₆ and a portion of the amino acid sequence of SEQ ID NO: 4 corresponding to the amino acid sequence of formula (1).

### Example 4-1: Preparation of AaOR9 Mutant (RX₆T) Expression Plasmid

A plasmid (pcDNA6.2) containing a sequence (base sequence: SEQ ID NO: 60) encoding an AaOR9 mutant (RX₆T) comprising the amino acid sequence of wild-type AaOR9 in which the 374th amino acid residue arginine (X₆ in formula (1)) was mutated to threonine (amino acid sequence: SEQ ID NO: 59) was prepared.

PCR primers designed to convert the 374th amino acid residue arginine (X₆ in formula (1)) in the amino acid sequence of wild-type AaOR9 to threonine (primer F:
CTCATATTTCACTCTTTTGAAAACGGTTTATAGCTAA (SEQ ID NO: 61) and primer R: TTAGCTATAAACCGTTTTCAAAAGAGTGAAATATGAG (SEQ ID NO: 62)) were synthesized. Using pcDNA6.2-AaOR9 as a template and using these primers, PCR was performed in the same manner as in the above Example to introduce mutation, thus obtaining an AaOR9 mutant (RX₆T) expression plasmid.

### Example 4-2: Preparation of AaOR9 Mutant (RX₆T + alpha) Expression Plasmid

A plasmid (pcDNA6.2) containing a sequence (base sequence: SEQ ID NO: 64) encoding an AaOR9 mutant (RX₆T + alpha) (amino acid sequence: SEQ ID NO: 63), which further contained additional multiple mutations in the AgOR9 mutant (RX₆T), was prepared. Fig. 4 shows the mutation sites. Specifically, using the AaOR9 (RX₆T) mutant expression plasmid (Example 4-1) as a PCR template and using each primer set for mutagenesis, mutations were sequentially introduced to prepare the plasmid.

### Test Example 4-2: Introduction of Expression Plasmid into Cells and Activity Measurement

The expression plasmid was introduced into cells in the same manner as in Test Example 1-2, and the activity was measured in the same manner as in Test Example 1-3. In this test example, skatole was used as a test substance.

Fig. 4 shows the results. The AaOR9 mutants obtained in Examples 4-1 to 4-2 were found to have higher chemical substance response activity than wild-type AaOR9.

### Test Example 5: Determination of Chemical Substance Response Activity of Other Insect Olfactory Receptors

The results of Test Examples 1 to 4 show that the chemical substance response activity of insect olfactory receptors can be enhanced by at least one of the following:
(A) X₃ is mutated to a branched-chain amino acid and/or X₄ is mutated to an uncharged polar amino acid;
(B) when the wild type has a sequence wherein X₅ and X₆ are both a positively charged polar amino acid, X₆ is mutated to an amino acid other than a positively charged polar amino acid; and
(C) when the wild type has a sequence wherein either X₁ or X₂ or both are a positively charged polar amino acid, X₁ is mutated to an uncharged polar amino acid and/or X₂ is mutated to a hydrophobic amino acid.

Further, using various insect olfactory receptors (such as AgOR28, AgOR47, AgOR11c, AgOR27a, AaOR5, AaOR31, AaOR72a, and AaOR110) a comparative test between the wild type and mutants thereof in chemical substance response activity was performed in the same manner as in Test Examples 1 to 4. The obtained results support the above rule.

## Claims

1. A mutant-insect olfactory receptor protein, comprising amino acid sequence B composed of amino acid sequence A containing a mutation, wherein
the amino acid sequence A is in a corresponding wild-type insect olfactory receptor protein and represented by formula (1) :
φ₇X₁X₂Z₁X₃X₄Z₂Z₃φ₆Uφ₅φ₄φ₃φ₂φ₁X₅X₆ wherein X₁ to X₆ and Z₁ to Z₃ represent amino acids derived from the amino acid sequence of the wild-type insect olfactory receptor, φ₁ represents a hydrophobic amino acid, φ₂ to φ₇ each independently represent an uncharged polar amino acid or a hydrophobic amino acid, and U represents an uncharged polar amino acid, and
the mutant-insect olfactory receptor protein satisfies at least one condition selected from the group consisting of the following conditions 1 to 3:
condition 1: in the amino acid sequence B,
X₃ and/or X₄ is substituted, and
X₃ is a branched-chain amino acid and/or X₄ is an uncharged polar amino acid,
condition 2: if X₅ and X₆ in the amino acid sequence A are both a positively charged polar amino acid, X₆ in the amino acid sequence B is an amino acid other than a positively charged polar amino acid, and
condition 3:
if either X₁ or X₂ or both in the amino acid sequence A are a positively charged polar amino acid,
in the amino acid sequence B,
X₁ and/or X₂ is substituted, and
X₁ is an uncharged polar amino acid and/or X₂ is a hydrophobic amino acid.

2. The mutant-insect olfactory receptor protein according to claim 1,
wherein
the mutant-insect olfactory receptor protein satisfies condition 1, and
X₃ represents isoleucine or leucine and/or X₄ represents serine or asparagine.

3. The mutant-insect olfactory receptor protein according to claim 1 or 2,
wherein
the mutant-insect olfactory receptor protein satisfies condition 2, and
X₆ represents an uncharged polar amino acid.

4. The mutant-insect olfactory receptor protein according to any one of claims 1 to 3,
wherein
the mutant-insect olfactory receptor protein satisfies condition 2, and
X₆ represents serine or threonine.

5. The mutant-insect olfactory receptor protein according to any one of claims 1 to 4,
wherein
the mutant-insect olfactory receptor protein satisfies condition 3, and
X₁ represents serine or glutamine and/or X₂ represents alanine.

6. The mutant-insect olfactory receptor protein according to any one of claims 1 to 5,
wherein
in condition 1, X₃ represents isoleucine or leucine and/or X₄ represents serine or asparagine,
in condition 2, X₆ represents serine or threonine, and
in condition 3, X₁ represents serine or glutamine and/or X₂ represents alanine.

7. The mutant-insect olfactory receptor protein according to any one of claims 1 to 6, which satisfies at least one condition selected from the group consisting of the following conditions 1A to 3A:
condition 1A: in the amino acid sequence B, X₃ is a branched-chain amino acid and/or X₄ is an uncharged polar amino acid,
condition 2A: in the amino acid sequence B, X₆ is an amino acid other than a positively charged polar amino acid, and
condition 3A: in the amino acid sequence B, X₁ is an uncharged polar amino acid and/or X₂ is a hydrophobic amino acid.

8. The mutant-insect olfactory receptor protein according to any one of claims 1 to 7, wherein the amino acid sequence A is a sequence present closer to the C-terminus of the amino acid sequence of the wild-type insect olfactory receptor protein.

9. The mutant-insect olfactory receptor protein according to any one of claims 1 to 8, wherein the wild-type insect olfactory receptor protein is a wild-type olfactory receptor protein of an insect in the superorder *Endopterygota.*

10. A polynucleotide comprising a coding sequence of the mutant-insect olfactory receptor protein of any one of claims 1 to 9.

11. A cell comprising the polynucleotide of Item 10.

12. A non-human animal comprising the cell of claim 11.

13. A chemical substance detection element comprising the mutant-insect olfactory receptor protein of any one of claims 1 to 9.

14. A chemical substance detection sensor comprising a lipid bilayer, a cell, or a non-human animal containing the cell, the lipid bilayer and the cell each containing the chemical substance detection element of claim 13.
